# EUROPÄISCHE PATENTSCHRIFT

(11) **EP 2 631 855 B1**
(45) Veröffentlichungstag und Bekanntmachung des Hinweises auf die Patenterteilung: **17.02.2016**
(21) Anmeldenummer: 12156381.1
(22) Anmeldetag: 21.02.2012
(51) Int. Cl.: G06Q 10/08, G06Q 50/22

(54) **Verfahren zum Auslösen einer durch ein Papierdokument autorisierten Aktion einer Vorrichtung**
Method for triggering an action of a device authorised by paper document
Procédé de déclenchement d'une action d'un dispositif automatisée par un document papier

(43) Veröffentlichungstag der Anmeldung: 28.08.2013
(73) Patentinhaber: CareFusion Germany 326 GmbH, 53539 Kelberg (DE)
(72) Erfinder: Wirtz, Michael, 54550 Daun-Neunkirchen (DE); Kohlhof, Andreas, 56414 Meudt (DE); Wiegland, Jens, 67578 Gimbsheim (DE)
(74) Vertreter: Zenz Patentanwälte Partnerschaft mbB

(56) Entgegenhaltungen:
- DE-U1-202009 011 994

## Beschreibung

Die Erfindung betrifft ein Verfahren zum Auslösen einer durch ein Papierdokument autorisierten Aktion einer Vorrichtung, wobei die Aktion ein eindeutiges Zuordnen einer Signatur zu dem Papierdokument voraussetzt.

Unter einer "Aktion einer Vorrichtung" soll im Rahmen dieser Erfindung eine mechanische oder elektromechanische Aktion oder eine Schaltaktion einer elektrischen Vorrichtung verstanden werden, beispielsweise das Einschalten einer Therapievorrichtung (wie beispielsweise einer Bestrahlung mit Licht vorgegebener Dauer) sowie eine Ausgabe einer Arzneimittelpackung oder eine Freigabe eines Zugriffs auf eine Arzneimittelpackung durch Entriegelung einer Sperre. Eine solche Aktion soll durch ein Papierdokument autorisiert sein. Ein "Papierdokument" soll hier irgendein bedrucktes und/oder beschriebenes Dokument aus einem bedruckbaren/beschreibbaren Material (beispielsweise Papier oder eine Kunststofffolie) sein, nicht aber ein elektronisches Dokument im Sinne einer bloßen Datei. "Autorisieren" bedeutet, dass das Papierdokument eine notwendige Voraussetzung sein soll, damit eine bestimmte Art einer solchen Aktion ausgeführt wird. Sofern die Aktion die Ausgabe einer Arzneimittelpackung bzw. die Freigabe eines Zugriffs einer Person auf eine bestimmte Arzneimittelpackung ist, kann das Papierdokument beispielsweise ein Rezept sein. Die Aktion der Vorrichtung soll zu ihrer Ausführung ein eindeutiges Zuordnen einer Signatur zu dem Papierdokument voraussetzen. Dies bedeutet im Falle eines Rezepts für eine Arzneimittelausgabe beispielsweise, dass die Abgabe des Arzneimittels nur dann ausgeführt werden soll, wenn dem Rezept in eindeutiger Weise eine Signatur zugeordnet worden ist. Herkömmlicherweise geschieht dies dadurch, dass das Rezept von einer autorisierten Person (beispielsweise einem Apotheker) mit einer Unterschrift abgezeichnet wird, womit der Apotheker zum Ausdruck bringt, dass das auf dem Rezept angegebene Arzneimittel abgegeben wurde. Die vorliegende Erfindung befasst sich mit einer Automatisierung dieses Vorgangs unter der Bedingung, dass sich die das Papierdokument durch Signatur üblicherweise abzeichnende autorisierte Person nicht an demjenigen Ort aufhält, an dem sich das Papierdokument befindet und die Aktion der Vorrichtung autorisieren soll, so dass eine unmittelbare Unterzeichnung des Papierdokuments nicht möglich ist.

Aus der Druckschrift DE 103 25 961 A1 ist ein Verfahren zum computergestützten Ausgeben eines Arzneimittels an einen Benutzer unter Verwendung eines automatisierten Apothekenlagers bekannt. Das aus der Druckschrift bekannte Verfahren geht von einem bekannten Verfahren zum Ausgeben eines Arzneimittels in einer Apotheke aus, in der Arzneimittelpackungen in einem automatisierten Apothekenregallager gelagert werden. Bei diesem Verfahren ist es beispielsweise bekannt, dass ein Apotheker von einem Kunden ein Rezept in Empfang nimmt und entsprechende Eingaben in eine Eingabevorrichtung (z.B. Computertastatur) des automatisierten Apothekenregallagers vornimmt, mit der er die auf dem Rezept enthaltenen Arzneimittelpackungen spezifiziert. Dann, nach Auslagerung der angeforderten Arzneimittelpackungen, nimmt der Apotheker ggf. eine Zahlung des Kunden in Empfang, woraufhin er die Arzneimittelpackungen an den Kunden übergibt.

Ausgehend davon beschreibt die Druckschrift ein Verfahren, das es dem Apotheker gestattet, mit Hilfe eines an einem ersten Ort, nämlich der Apotheke, installierten automatisierten Arzneimittellagers (auch Kommissionierautomat genannt) und eines mit dem Lager verbundenen Ausgabe-Terminals über ein Datenfernübertragungsnetzwerk (beispielsweise das Internet) von einem entfernten Ort aus (beispielsweise einem Heimarbeitsplatz) die Ausgabe des Medikaments ferngesteuert vorzunehmen. Bei dem dort beschriebenen Verfahren nimmt der Kunde zunächst durch Betätigung einer Eingabeeinrichtung an dem Ausgabe-Terminal eine Eingabe vor (beispielsweise durch Drücken einer Ruftaste), welche anzeigt, dass der Kunde ein Arzneimittel anfordern möchte. Nach Erfassen dieser Eingabe wird über einen Datenfernübertragungskanal ein Rufsignal an eine entfernt angeordnete Datenkommunikationseinrichtung übermittelt, beispielsweise an den von einem Apotheker bedienten Computer. Anschließend wird eine bidirektionale Datenkommunikationsverbindung zwischen dem Computer des Apothekers und einem Computer des Ausgabe-Terminals hergestellt. Beide Computer sind mit Kameras, Mikrofonen, Bildschirmen und Lautsprechern ausgestattet, so dass über diesen Kanal ein persönlicher Dialog zwischen dem Apotheker und dem Kunden vorgenommen werden kann, in dessen Rahmen der Apotheker den Kunden berät. Im Verlauf des Dialogs legt der Kunde beispielsweise ein Rezept in eine am Ausgabe-Terminal vorhandene Rezeptleseeinrichtung ein. Ein Bild des gelesenen Rezepts kann dann zum Computer des Apothekers übertragen werden. Der Apotheker prüft das Rezept, führt den Dialog mit dem Kunden weiter und entscheidet dann durch entsprechende Eingaben, welche Arzneimittelpackungen aus dem automatisierten Regallager zu dem Ausgabe-Terminal transportiert werden sollen. Dort landen die auszugebenden Arzneimittelpackungen in einem Ausgabeplatz, auf den der Kunde zunächst noch keinen Zugriff hat. Anschließend prüft der Apotheker, ob die richtigen Arzneimittel am Ausgabeplatz liegen. Ferner prüft er ggf., ob der Kunde über an dem Ausgabe-Terminal befindliche Bezahlvorrichtungen eine zur Ausgabe der Arzneimittelpackungen erforderliche Zahlung vorgenommen hat. Die Bezahlvorrichtungen umfassen beispielsweise einen Geldscheineinzug, einen Münzeinwurf und/oder ein Geldkartenlesegerät. Wenn schließlich alle Erfordernisse für die Ausgabe der Arzneimittelpackungen erfüllt sind, gibt der Apotheker ferngesteuert den Zugriff auf den Ausgabeplatz frei, so dass der Kunde die Arzneimittelpackungen entnehmen kann. Die Druckschrift offenbart auch, dass die Erfassung der auszugebenden Arzneimittelpackungen beispielsweise durch automatisiertes Lesen des in Papierform vorliegenden Rezepts vorgenommen werden kann. Allerdings schweigt die Druckschrift über den Verbleib des Rezepts nach Ausgabe der Arzneimittel.

Aus der DE 20 2009 011 994 U1 ist ferner ein Ausgabesystem für automatisierte Ausgaben von Waren bekannt, bei welchem ein eingelegtes Rezept mit einem Aufdruck versehen wird, wobei der Aufdruck einen Code, z.B. eine elektronische Signatur, aufweist.

Bei der Abgabe eines rezeptpflichtigen Arzneimittels ist ein Apotheker in Deutschland gesetzlich dazu verpflichtet, zum Zeitpunkt der Abgabe der auf dem Rezept verzeichneten Arzneimittelpackungen eventuelle Änderungen auf dem Rezept zu vermerken und das Rezept abzuzeichnen. Dies ist bei der bekannten Fernabgabevorrichtung selbstverständlich nicht möglich, da der Apotheker keinen unmittelbaren Zugriff auf das Rezept hat. Daher wäre es wünschenswert, ein Verfahren zur ferngesteuerten Abgabe von Arzneimittelpackungen an einem Abgabe-Terminal zu schaffen, das die vom Gesetzgeber geforderte Sicherheit bei der Abgabe von rezeptpflichtigen Arzneimitteln schafft und es dem Apotheker gestattet, seine Signatur in einer eindeutigen und nachträglich nicht manipulierbaren Weise dem Papierdokument (Rezept) zuzuordnen.

Ausgehend von dem eingangs genannten Verfahren ist es somit Aufgabe der Erfindung, ein Verfahren zum Auslösen einer durch ein Papierdokument autorisierten Aktion einer Vorrichtung zu schaffen, bei dem die Aktion ein eindeutiges Zuordnen einer Signatur zu dem Papierdokument voraussetzt, das es ermöglicht, dass sich das Papierdokument und die signierende autorisierte Person an räumlich getrennten Orten befinden.

Diese Aufgabe wird erfindungsgemäß durch ein Verfahren mit den Merkmalen des Anspruchs 1 gelöst.

Bei dem Verfahren zum Auslösen einer durch ein Papierdokument autorisieren Aktion einer Vorrichtung, bei dem die Aktion ein eindeutiges Zuordnen einer Signatur zu dem Papierdokument voraussetzt, wird zunächst an einem ersten Ort, an dem die Aktion ausgeführt werden soll, das Papierdokument in eine Aufnahme eingelegt, von einer Aufbringvorrichtung ein erster eindeutiger Identifizierer auf das Papierdokument unlösbar aufgebracht und ein elektronisches Abbild des Papierdokuments erzeugt, wobei dem ersten Identifizierer ein zweiter Identifizierer eindeutig zugeordnet wird und aus dem elektronischen Abbild ein elektronisches Dokument erzeugt und mit dem zweiten Identifizierer verbunden wird. Dabei wird das elektronische Abbild des Papierdokuments erstellt, nachdem der erste Identifizierer auf dem Papierdokument unlösbar aufgebracht worden ist, wobei das elektronische Abbild, aus dem das elektronische Dokument erzeugt wird, ein Abbild zumindest eines Teils des Papierdokuments mit dem aufgebrachten ersten Identifizierer umfasst. Diese Vorgehensweise erhöht die Sicherheit der Zuordnung der Identifizierer zu dem Papierdokument, da sie es gestattet, dass das Abbild des elektronischen Dokuments, das an dem zweiten, entfernten Ort der zum Signieren autorisierten Person angezeigt wird, einerseits erkennen lässt, ob der erste Identifizierer korrekt auf dem Papierdokument aufgebracht worden ist, und andererseits einen Vergleich des ersten Identifizierers, sofern dieser auf dem Abbild lesbar ist, mit dem mit dem elektronischen Dokument verbundenen zweiten Identifizierer, beispielsweise dem Dateinamen, ohne weiteres gestattet. Der erste Ort, an dem die Aktion ausgeführt werden soll, ist beispielsweise der Ort, an dem sich ein Arzneimittelausgabeautomat befindet oder auch der Ort, an dem eine Therapiemaßnahme von einem Automaten durchgeführt werden soll. Vorzugsweise ist der erste Ort derjenige, an den sich ein Arzneimittelausgabe-Terminal befindet, das mit einer automatisierten Lagervorrichtung (beispielsweise einem Kommissionierautomaten) gekoppelt ist. Der auf das Papierdokument unlösbar aufgebrachte erste eindeutige Identifizierer ist beispielsweise eine aufgedruckte Nummer, ein aufgedruckter Zeichencode, Barcode oder 2D-Code oder auch ein aufgeklebtes RFID-Transponder- oder Speicher-Chip. Dementsprechend ist die Aufbringvorrichtung beispielsweise ein Drucker oder auch eine Aufklebevorrichtung zum Aufkleben eines Tags oder eines RFID-Chips. Das elektronische Abbild des Papierdokuments ist beispielsweise eine Grafikdatei, z. B. in einem PDF-Format. Der dem ersten Identifizierer eindeutig zugeordnete zweite Identifizierer ist beispielsweise ein Dateiname, der einen Code des ersten Identifizierers umfasst. Das Verbinden des zweiten Identifizierers mit dem elektronischen Dokument erfolgt beispielsweise einfach dadurch, dass das elektronische Dokument einen den Code enthaltenden Dateinamen erhält.

Bei dem erfindungsgemäßen Verfahren wird dann ein Abbild des elektronischen Dokuments auf einer Ausgabevorrichtung einer an einem zweiten, entfernten Ort befindlichen Datenverarbeitungseinrichtung einer zum Signieren autorisierten Person angezeigt und wird das elektronische Dokument von der autorisierten Person durch eine vorgegebene Eingabe in eine Eingabevorrichtung elektronisch signiert. Das Abbild des elektronischen Dokuments kann das gesamte Dokument oder auch nur einen Teil des Dokuments wiedergeben. Die Ausgabevorrichtung ist beispielsweise ein Computerbildschirm, und die an einem zweiten, entfernten Ort befindliche Datenverarbeitungseinrichtung ist beispielsweise ein Computer, der sich an einem Heimarbeitsplatz des Apothekers befindet. Die vorgebebene Eingabe, die von der autorisierten Person vorgenommen wird, um das elektronische Dokument elektronisch zu signieren, ist beispielsweise die Eingabe einer PIN oder eines Fingerabdrucks, ein Netzhautscan oder das Lesen eines elektronischen Personalausweises. Die zugehörige Eingabevorrichtung ist beispielsweise eine Computertastatur, eine Chipkartenleseeinrichtung, ein Fingerprint-Scanner oder eine Kamera.

In einem nächsten Schritt des erfindungsgemäßen Verfahrens wird das mit dem zweiten Identifizierer verbundene und signierte elektronische Dokument in einem abgesicherten Speicher gespeichert. Sobald das signierte elektronische Dokument gespeichert worden ist, wird die autorisierte Aktion durch die Vorrichtung ausgeführt.

Das erfindungsgemäße Verfahren ersetzt das Aufbringen einer manuellen Unterschrift auf dem Papierdokument, welche Voraussetzung für das Ausführen der autorisierten Aktion der Vorrichtung ist, durch das unlösbare Aufbringen des ersten Identifizierers auf dem Papierdokument und die eindeutige Zuordnung des ersten Identifizierers zu dem mit dem elektronischen Dokument (untrennbar) verbundenen zweiten Identifizierer. Das Verfahren gestattet somit die Weiterverwendung des herkömmlich verwendeten Papierdokuments, das eine bestimmte Aktion autorisiert, insbesondere die Weiterverwendung eines herkömmlichen Papierrezepts, mit welchem der Aussteller (das heißt der Arzt) bestimmte Arzneimittel verschreibt und somit deren Übergabe an den Kunden (Patienten) autorisiert. Dies ermöglicht die Ausgabe rezeptpflichtiger Arzneimittelpackungen an einer Apotheke mit einem Arzneimittelausgabe-Terminal, welches mit einem automatisierten Arzneimittellager gekoppelt ist, und die Fernsteuerung dieser Ausgabe durch einen an einem entfernten Ort handelnden Apotheker. Das Verfahren sichert die rechtssichere Abgabe von rezeptpflichtigen Arzneimitteln über das Arzneimittelausgabe-Terminal durch Einsatz einer qualifizierten elektronischen Signatur und durch Sicherstellung, dass die Abgabe des Arzneimittels an den Kunden erst nach Abzeichnung des Rezepts durch eindeutige Zuordnung des aufgebrachten ersten Identifizierers zu einem mit dem signierten elektronischen Dokument verbundenen zweiten Identifizierer erfolgt.

Eine bevorzugte Ausführungsform des erfindungsgemäßen Verfahrens ist dadurch gekennzeichnet, dass der erste Identifizierer einen alphanumerischen Code umfasst, wobei der zweite Identifizierer dem ersten Identifizierer eindeutig zugeordnet und mit dem elektronischen Dokument verbunden wird, indem ein den alphanumerischen Code enthaltender Dateiname des elektronischen Dokuments als zweiter Identifizierer verwendet wird. Dies vereinfacht die Zuordnung der elektronischen Dokumente in dem abgesicherten Speicher zu den den ersten Identifizierer tragenden Papierdokumenten.

Eine Weiterbildung des Verfahrens ist dadurch gekennzeichnet, dass vor dem Aufbringen des ersten eindeutigen Identifizierers auf das Papierdokument im Schritt a) das Papierdokument in eine Aufnahme eingelegt und danach der Zugriff auf das Papierdokument gesperrt wird, wobei dann, wenn das Verfahren vor dem Ausführen der Aktion oder auch nur eines Teils der Aktion abgebrochen, der Zugriff auf das Papierdokument zur Entnahme wieder freigegeben wird, anderenfalls nach vollständigem oder teilweisem Ausführen der Aktion das Papierdokument in einer gegen unbefugten Zugriff gesicherten Ablage abgelegt wird. Durch diese Weiterbildung wird sichergestellt, dass das Papierdokument nicht erneut zum Auslösen der autorisierten Aktion verwendet werden kann, wenn diese bereits einmal ausgeführt worden ist. Bei vollständigem Ausführen der Aktion, beispielsweise bei vollständiger Ausgabe der auf dem Rezept verzeichneten Arzneimittel, wird das Papierdokument (Rezept) einbehalten und gegen unbefugten Zugriff gesichert. Bei teilweiser Ausführung der Aktion des Papierdokuments, beispielsweise bei einem teilweisen Ausgeben der auf dem Rezept verzeichneten Arzneimittel, wird dieser ausgeführte Teil der Aktion, beispielsweise die ausgegebenen Arzneimittel, auf dem Papierdokument aufgedruckt und dann das Papierdokument (Rezept) einbehalten und gegen unbefugten Zugriff gesichert. (Bei einer alternativen Ausführungsform könnte nach dem Aufdrucken des ausgeführten Teils der Aktion das Papierdokument auch wieder entnommen werden.) Das Papierdokument kann schließlich wieder entnommen werden, wenn die Aktion nicht (auch nicht teilweise) ausgeführt wurde, das heißt das Verfahren vor dem Ausführen der Aktion abgebrochen wurde. Bei einer Weiterbildung dieser Ausführungsform des erfindungsgemäßen Verfahrens wird das Papierdokument vor dem Ablegen in die gegen unbefugten Zugriff gesicherte Ablage mit einem das vollständige Ausführen der Aktion bzw. den ausgeführten Teil der Aktion kennzeichnenden Aufdruck versehen. Beispielsweise wird ein Rezept mit einem die vollständige Abgabe der darauf verzeichneten Arzneimittel kennzeichnenden Aufdruck versehen.

Bei einer bevorzugten Ausführungsform des erfindungsgemäßen Verfahrens enthält das Papierdokument Angaben, die die Art der autorisierten Aktion definieren. In diesem Fall wird vor dem Schritt a) ein erstes elektronisches Abbild des Papierdokuments erstellt, beispielsweise das Papierdokument gescannt und in eine Bilddatei konvertiert. Auf der Basis des ersten elektronischen Abbilds wird dann die von den Angaben definierte Art der Aktion bestimmt. Anschließend wird ermittelt, ob es möglich ist, die definierte Art der Aktion (oder auch einen Teil der Aktion) an dem ersten Ort auszuführen. Nur dann, wenn dies der Fall ist, wird das Verfahren mit dem Schritt a) fortgesetzt, wobei das im Schritt a) erzeugte elektronische Abbild, aus dem das elektronische Dokument erzeugt wird, ein zweites elektronisches Abbild ist. Bei dieser Ausführungsform des erfindungsgemäßen Verfahrens wird somit vorzugsweise zunächst ein erstes Abbild des Papierdokuments erzeugt, anhand dessen geprüft wird, ob die darauf definierte Art der Aktion ausgeführt werden kann. Anschließend wird nach dem Aufbringen des ersten Identifizierers auf das Papierdokument ein zweites elektronisches Abbild erzeugt, das für die Signaturzwecke weiterverwendet wird. Durch diese Weiterbildung kann ein Bedrucken des Papierdokuments für den Fall vermieden werden, dass es beispielsweise nicht möglich ist, die auf dem Papierdokument gekennzeichnete autorisierte Aktion, beispielsweise die Ausgabe der darauf verzeichneten Arzneimittel, auszuführen.

Eine Weiterbildung dieses zuletzt genannten Verfahrens ist dadurch gekennzeichnet, dass auf der Basis des ersten elektronischen Abbilds die von den Angaben definierte Art der Aktion bestimmt wird, indem die Angaben mittels eines Texterkennungsprogramms ermittelt werden und automatisch ermittelt wird, ob es möglich ist, die definierte Art der Aktion auszuführen. Im Falle der Verwendung eines Rezepts zur automatischen Ausgabe von Arzneimitteln bedeutet dies, dass zunächst automatisch ermittelt wird, welche Arzneimittel auf dem Rezept verzeichnet sind und dann bestimmt wird, ob deren Ausgabe möglich ist.

Bei einer alternativen Ausführungsförm, die im Fall einer Fernsteuerung der Arzneimittelausgabe durch einen Apotheker bevorzugt ist, wird auf der Basis des ersten elektronischen Abbilds die von den Angaben definierte Art der Aktion bestimmt, indem das Abbild sowie Angaben zu ausführbaren Aktionen auf der Ausgabevorrichtung (z. B. Bildschirm) der an dem zweiten Ort befindlichen Datenverarbeitungseinrichtung der autorisierten Person angezeigt werden und die autorisierte Person in Abhängigkeit davon die definierte Art der Aktion durch entsprechende Eingaben vorgibt. Im Falle eines Rezepts für eine Arzneimittelausgabe bedeutet dies, dass auf dem Bildschirm des entfernt tätigen Apothekers einerseits das erste elektronische Abbild des Rezepts angezeigt wird, andererseits angezeigt wird, welche Arzneimittel vorrätig sind und an dem ersten Ort ausgegeben werden können. Der Apotheker vergleicht diese Angaben und gibt dann die Ausgabe der vorhandenen Arzneimittelpackungen frei. Er kann dabei auch für den Fall, dass das auf dem Rezept verzeichnete Arzneimittel nicht vorrätig ist, ggf. die Ausgabe eines alternativen Arzneimittels freigeben.

Eine bevorzugte Ausführungsform des erfindungsgemäßen Verfahrens ist dadurch gekennzeichnet, dass das Papierdokument einen Arzneimittel-Rezept und die autorisierte Aktion eine Abgabe von auf dem Rezept verzeichneten Arzneimittelpackungen an einen Kunden an dem ersten Ort durch eine Abgabevorrichtung ist, wobei das Abbild des elektronischen Dokuments an dem zweiten, entfernten Ort, einem Apotheker als der zum Signieren berechtigten Person angezeigt wird. Unter einem "Apotheker" soll hier auch eine sonstige zur Abzeichnung eines Rezepts befugte Person verstanden werden.

Diese zuletzt genannte Ausführungsform ist vorzugsweise dadurch gekennzeichnet, dass im Schritt a)
a1) von der Aufnahmevorrichtung an dem ersten Ort ein erstes elektronisches Abbild des Rezepts erzeugt und auf der Ausgabevorrichtung der an dem zweiten, entfernten Ort befindlichen Datenverarbeitungseinrichtung dem Apotheker angezeigt wird,
a2) anschließend geprüft wird, ob eine Abgabe der auf dem Rezept verzeichneten Arzneimittelpackungen durch einen an dem ersten Ort befindlichen Abgabeautomaten möglich ist,
a3) sofern diese Abgabe möglich ist, durch eine entsprechende Eingabe des Apothekers ein Transport der abzugebenden Arzneimittelpackungen zu einem Ausgabefach angewiesen wird, wobei der Zugriff des Kunden auf das Ausgabefach gesperrt wird,
a4) dann mittels Erfassungseinrichtungen an dem ersten Ort geprüft wird, ob weitere Voraussetzungen für die Abgabe der Arzneimittelpackungen erfüllt sind,
a5) sofern die weiteren Voraussetzungen erfüllt sind, von der Aufbringvorrichtung der erste eindeutige Identifizierer auf das Rezept aufgebracht und von der Aufnahmevorrichtung ein zweites elektronisches Abbild des Rezepts erzeugt wird, und
a6) aus dem zweiten elektronischen Abbild das elektronische Dokument erzeugt und mit dem zweiten Identifizierer verbunden wird, und
dass im Schritt d) der Zugriff des Kunden auf das Ausgabefach freigegeben wird. Bei dieser Weiterbildung ist die Abgabesicherheit weiter erhöht, indem dem Kunden der Zugriff auf das Ausgabefach erst dann ermöglicht wird, wenn weitere Voraussetzungen für die Ausgabe der Arzneimittelpackungen erfüllt sind und die elektronische Signatur ausgeführt worden ist.

Das Verfahren ist vorzugsweise dadurch gekennzeichnet, dass im Schritt a4) mittels einer auf das Ausgabefach gerichteten Kamera ein Bild der in das Ausgabefach transportierten Arzneimittelpackungen erzeugt und als weitere Voraussetzung für die Abgabe der Arzneimittelpackungen anhand des Bildes geprüft wird, ob sich die richtigen Arzneimittelpackungen im Ausgabefach befinden. Diese Weiterbildung erhöht die Abgabesicherheit, da der Apotheker vor der Freigabe des Zugriffs abschließend prüfen kann, ob der Ausgabeautomat die richtigen Arzneimittelpackungen ausgelagert hat.

Bei einer anderen bevorzugten Weiterbildung des zuletzt genannten Verfahrens wird im Schritt a4) eine Aufforderung für einen Bezahlvorgang an den Kunden ausgegeben und als weitere Voraussetzungen für die Abgabe der Arzneimittelpackungen geprüft, ob der Bezahlvorgang erfolgreich abgeschlossen wurde. Vorteilhafterweise wird hier der Zugriff auf das Ausgabefach zusätzlich von einem erfolgreichen Bezahlvorgang abhängig gemacht.

Bei einer vorteilhaften Weiterbildung wird im Schritt d) geprüft, ob sämtliche Arzneimittelpackungen (nach Freigabe des Zugriffs) vollständig aus dem freigegebenen Ausgabefach entnommen worden sind, wobei dann, wenn dies nicht der Fall ist, ein Signal an den Kunden ausgegeben wird. Dieser wird aufgefordert, für eine vollständige Entnahme der Arzneimittelpackungen zu sorgen.

Eine andere Weiterbildung dieses Verfahrens ist dadurch gekennzeichnet, dass im Schritt d) geprüft wird, ob sämtliche Arzneimittelpackungen vollständig aus dem freigegebenen Ausgabefach entnommen worden sind, wobei dann, wenn dies der Fall ist, das Papierdokument mit einem das vollständige Ausführen der autorisierten Aktion kennzeichnenden Aufdruck versehen wird, andernfalls das Papierdokument mit einem die unvollständige Entnahme der Arzneimittelpackungen kennzeichnenden Aufdruck versehen wird. Die nicht entnommenen Arzneimittelpackungen werden dann beispielsweise zurück in das Arzneimittellager transportiert. Auf diese Weise wird auch die unvollständige Entnahme der ausgelagerten Arzneimittelpackungen dokumentiert und dem eingelösten Rezept zugeordnet.

Vorteilhafte und/oder bevorzugte Weiterbildungen der Erfindung sind in den Unteransprüchen gekennzeichnet.

Im Folgenden wird die Erfindung anhand eines in den Zeichnungen dargestellten bevorzugten Ausführungsbeispiels näher erläutert. In den Zeichnungen zeigen

Figur 1 eine schematische Darstellung der zur Ausführung des erfindungsgemäßen Verfahrens verwendeten miteinander gekoppelten Vorrichtungen und

Figuren 2A und 2B eine schematische Darstellung der Prozessabläufe einer bevorzugten Ausführungsform des erfindungsgemäßen Verfahrens.

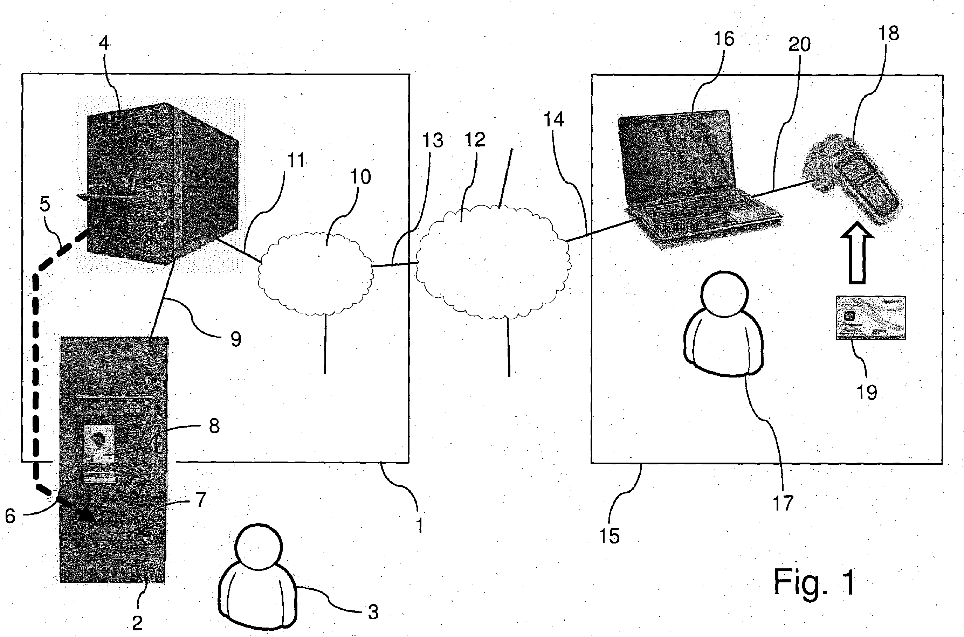

Figur 1 zeigt eine schematische Darstellung der zur Ausführung des erfindungsgemäßen Verfahrens verwendeten Vorrichtungen. An einem ersten Ort befinden sich die Räumlichkeiten 1 einer Apotheke, die durch ein Rechteck dargestellt sind. Ein Arzneimittelausgabe-Terminal 2 ist so angeordnet, dass es auch bei geschlossenen Räumlichkeiten 1 der Apotheke von einer Frontseite her zugänglich ist. Die Rückseite des Arzneimittelausgabe-Terminals befindet sich innerhalb der Räumlichkeiten 1 der Apotheke. Diese Art der Anordnung des Terminals 2 ist in Figur 1 angedeutet, indem das Terminal 2 auf der Umgrenzungslinie 1 dargestellt ist. Das Terminal 2 kann beispielsweise in einem Vorraum der Apotheke aufgestellt sein. Ein Kunde 3, der die Ausgabe eines Arzneimittels, insbesondere eines rezeptpflichtigen Arzneimittels, wünscht, tritt dann vor die Frontseite des Terminals 2. Das Terminal 2 umfasst alle erforderlichen Einrichtungen, die dem Kunden 3 einen Dialog mit einem Apotheker, das Vorlegen eines Rezepts, das Bezahlen eines geforderten Betrags und das Entnehmen der Arzneimittelpackungen ermöglichen. Insbesondere umfasst das Terminal 2 ein Ausgabefach 7, welches durch eine verriegelbare Tür (beispielsweise eine Klappe oder ein Rolladen) verschlossen ist, in welches die auszugebenden Arzneimittelpackungen transportiert werden und aus welchem der Kunde 3 die gewünschten Arzneimittelpackungen (nach Öffnen der Tür) entnehmen kann. Das Arzneimittelausgabe-Terminal 2 umfasst ferner einen Einzugsschacht 6 für Rezepte, in den der Kunde 3 ein Rezept einführen kann. Ferner weist das Terminal 2 einen Rufknopf zum Initiieren einer gewünschten Arzneimittelausgabe, einen Touchscreen 8 zur Wiedergabe von Aufforderungen an den Kunden und zur Eingabe durch den Kunden, sowie Mikrofon, Lautsprecher und eine Kamera zum Durchführen eines Dialogs mit einem Apotheker auf. Schließlich umfasst das Terminal 2 Bezahlvorrichtungen, wie beispielsweise einen Münzeinwurfschacht, einen Geldscheineinzug, einen Kartenleser mit einen PIN-Eingabefeld, einen Bondrucker und eine Münzrückgabe auf.

Im Inneren des Terminals befinden sich, für den Kunden nicht zugänglich, weitere Komponenten, welche eine Datenverarbeitungseinrichtung (Server) für das Terminal 2, eine Rezeptscanner/- Druckerkombination, ein Sammelfach für abgelegte Rezepte sowie Bezahlvorrichtungen, wie Münzprüfer, Münzsammelbehälter, Wechselgeldbehälter und Geldscheinprüfer, umfassen. Das Terminal 2 besitzt ein massives Metallgehäuse. Der Zugang zu seinem Inneren ist durch ein Schloss an der Rückseite gesichert, wobei sich der Schlüssel üblicherweise im Besitz des Apothekers befindet. Ein Zugang zum Gehäuseinneren ist von der Frontseite her nicht möglich.

Der in dem Terminal 2 enthaltene Server ist über ein eigenes Netzwerk mit weiteren Komponenten des Terminals und mit einem Arzneimittelkommissionierautomaten 4 gekoppelt, wobei die Kopplung zwischen dem Terminal 2 und dem Kommissionierautomaten 4 durch die Leitung 9 in Figur 1 dargestellt ist. Die Verbindung zwischen dem Terminal 2 und dem Kommissionierautomaten 4 wird üblicherweise durch ein in der Apotheke verlegtes Kabel zwischen einem Router des Kommissionierautomaten 4 und einem Switch des Terminals 2 hergestellt. Zugriffe auf dieses Netzwerk des Kundenterminals von außen sind nur im Rahmen der in einer Firewall des Routers des Kommissionierautomaten 4 hinterlegten Regeln möglich (beispielsweise über eine gesicherte Verbindung oder ein VPN).

Der Kommissionierautomat 4 enthält Lagerregale zum Lagern von Arzneimittelpackungen, wobei die Lagerregale innerhalb eines Gehäuses beispielsweise so angeordnet sind, dass zwischen ihnen eine Gasse gebildet wird. In der Gasse zwischen den Lagerregalen bewegt sich ein Regalbediengerät, welches die Packungen auf den Regalböden ablegen und von den Regalböden entnehmen kann. Auch in den Zeiten, in denen die Apotheke geöffnet ist, dient der Kommissionierautomat zum Ein- und Auslagern von Medikamentenpackungen, wobei die ausgelagerten oder ausgegebenen Arzneimittelpackungen üblicherweise zu einem Ausgabeplatz in der Offizin der Apotheke transportiert werden. Das Regalbediengerät, welches die Packungen von den Regalböden entnommen hat, wirft diese beispielsweise in einen Ausgabeschacht, über welchen sie dann über Rutschen oder Transportbänder zum Abgabeplatz in der Offizin transportiert werden. In den Zeiten jedoch, in denen die Apotheke personell nicht besetzt ist, wird zum Ausführen des erfindungsgemäßen Verfahrens der Kommissionierautomat 4 über eine Transportvorrichtung 5 mit dem Arzneimittelausgabe-Terminal 2 gekoppelt. Das Regalbediengerät des Kommissionierautomaten 4 legt die Packungen auf der Transportvorrichtung 5 ab, die die Packungen schließlich zum Ausgabefach 7 des Terminals 2 transportiert. Die Transportvorrichtung 5 umfasst hierbei Förderbänder und/oder Rutschen oder andere Vorrichtungen, die beispielsweise auf einer Rohrposttechnik basieren. Der Kommissionierautomat 4 enthält einen Steuerungscomputer. Alle,Komponenten des Kommissionierautomaten 4 sind wiederum durch ein Anlagennetzwerk miteinander verbunden, und dieses Anlagennetzwerk des Kommissionierautomaten 4 kann über eine Leitung 11 mit einem bestehenden Computernetzwerk 10 der Apotheke gekoppelt sein. An das Apothekennetzwerk 10 können beispielsweise diverse Computer angekoppelt sein, die in der Apotheke aufgestellt sind.

Das Apothekennetzwerk ist wiederum über eine Kommunikationsleitung 13, die beispielsweise das öffentliche Fernsprechnetz umfasst, mit dem Internet 12 verbunden. Figur 1 zeigt auf der rechten Seite einen Kasten 15, der einen Heimarbeitsplatz eines Apothekers 17 kennzeichnet. Dort befindet sich ein Computer 16, der über die Leitung 14 ebenfalls mit dem Internet 12 gekoppelt ist. Darüber hinaus ist der Computer 16 über eine Verbindung 20 mit einem Kartenlesegerät 18 verbunden. In das Kartenlesegerät 18 kann eine Signaturkarte 19 eingelegt werden. Selbstverständlich können auch mehrere Heimarbeitsplätze von verschiedenen Apothekern an verschiedenen Orten über das Internet 12 mit dem Computernetzwerk 10 der Apotheke oder mit dem Anlagennetzwerk des Kommissionierautomaten 4 gekoppelt sein. Anstelle einer Kopplung über das Internet 12 ist auch eine Kopplung über eine beliebige andere bidirektionale Datenkommunikationsverbindung denkbar. Beispielsweise kann der Computer 16 auch über ein Mobilfunknetz mit dem Netzwerk 10 der Apotheke oder dem Anlagennetzwerk des Kommissionierautomaten 4 verbunden werden.

Auf den Datenverarbeitungseinrichtungen der in Figur 1 gezeigten diversen Komponenten des Systems, nämlich auf den Datenverarbeitungseinrichtungen des Arzneimittelausgabe-Terminals 2, des Kommissionierautomaten 4 und des Computers 16 laufen diverse Programme, die zum Ausführen bestimmter Schritte des erfindungsgemäßen Verfahrens dienen. Auf der als Server bezeichneten Datenverarbeitungseinrichtung des Terminals 2 gibt es eine als "Application Server" bezeichnete Softwarekomponente, die die Ansteuerung der in dem Terminal 2 enthaltenen Hardwarekomponenten und die gesamte Vorgangssteuerung übernimmt und über einen eingebundenen Webserver eine Oberfläche für die Verwaltung und Wartung des Systems durch den Apotheker von dessen Computer aus bereitstellt. Es gibt ferner eine Benutzerschnittstelle, über die ein Kunde 3 das Terminal 2 bedient. Eine Datenbank dient zur Speicherung sämtlicher Vorgangsdaten. Protokolle, Log-Dateien und signierte Rezepte werden auf einer Festplatte (oder mehreren) des Servers gespeichert. Eine Softwarekomponente ermöglicht den Aufbau einer Videokonferenz zwischen dem Terminal 2 und dem Computer 16 des Apothekers 17. Darüber hinaus enthält der Server noch Softwarekomponenten zur Erhöhung der Datensicherheit, wie beispielsweise eine Firewall. Der Rechner des Kommissionierautomaten 4 enthält die Steuerungs- und Verwaltungssoftware zum Ansteuern der Komponenten des Kommissionierautomaten und zur Lagerverwaltung. Darüber hinaus enthält der Rechner die zum Herstellen der genannten Verbindungen erforderliche Kommunikationshardware und -software. Der Rechner 16 am Apotheker-Heimarbeitsplatz enthält neben dem Betriebssystem und diversen Anwendungsprogrammen auch ein Apotheker-Anwendungsprogramm, mit der der Apotheker die Arzneimittelausgabe und den Verkaufsvorgang steuern und kontrollieren kann. In dieses Apotheker-Anwendungsprogramm sind Softwarekomponenten eingebettet, die für die elektronische Signatur und die Videokonferenz verwendet werden. Zur Kommunikation mit dem Kartenlesegerät 18 enthält der Computer 16 die erforderlichen Kartenleser-Treiber. Für die Erstellung und Prüfung einer qualifizierten elektronischen Signatur enthält der Rechner 16 eine Signatursoftware. Der gesamte Signäturprozess wird ausschließlich von der Signatursoftware kontrolliert und schließt die Schritte des Öffnens der zu signierenden Datei, des Führens des Anwenders durch den Signaturprozess und der Darstellung der zu signierenden Inhalte, des Ansteuerns des Kartenlesers, der Durchführung der Signatur und des Speicherns der signierten Datei ein.

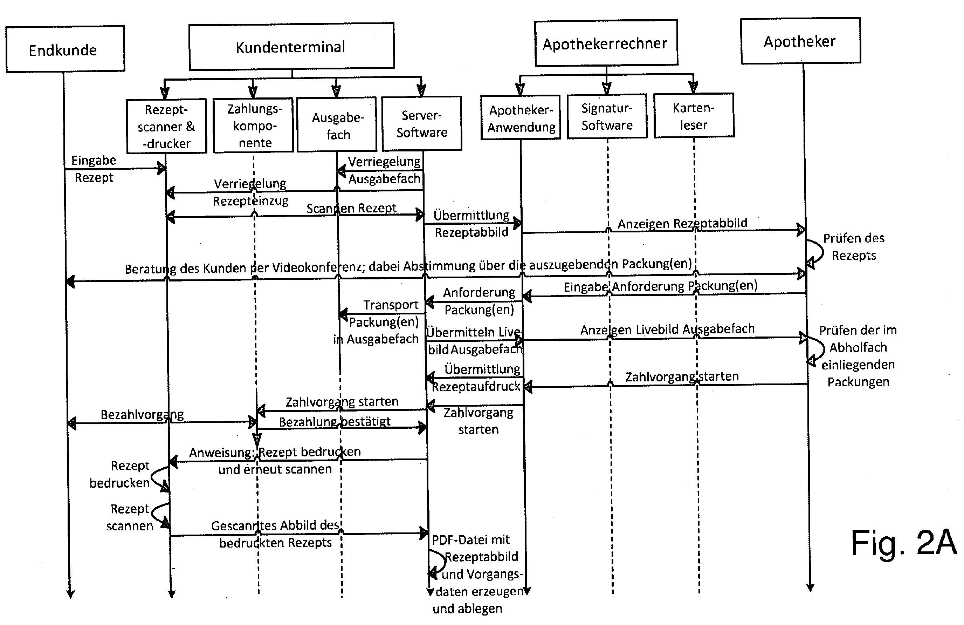

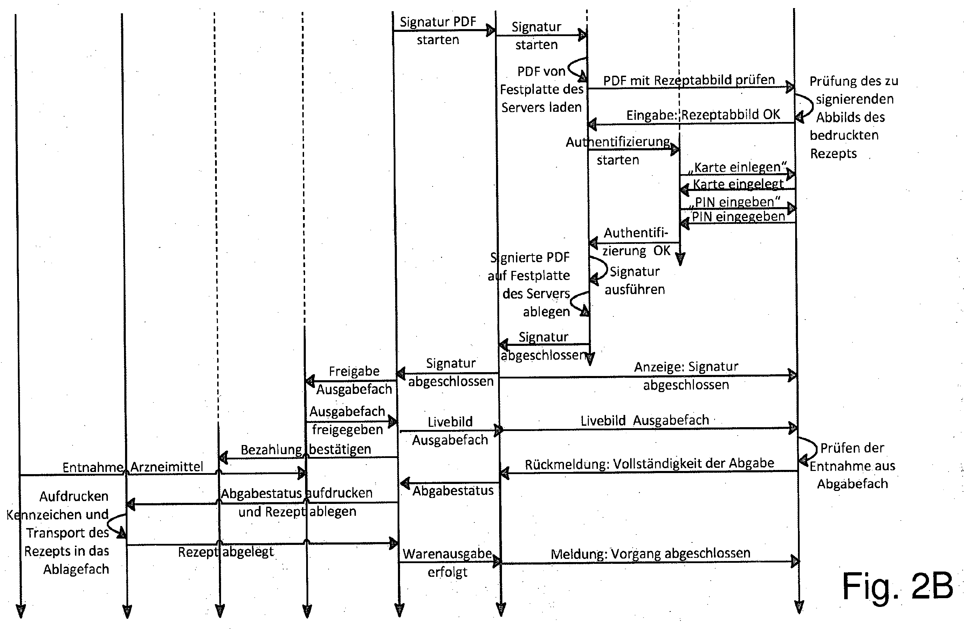

Nachfolgend wird anhand der Figuren 2A und 2B ein bevorzugtes Verfahren zur Ausgabe rezeptpflichtiger Arzneimittel über das ferngesteuerte Arzneimittelausgabe-Terminal 2 beschrieben.

Das Verfahren beginnt damit, dass der vor dem Terminal 2 stehende Kunde 3 sein Rezept in den Einzugsschacht 6 für Rezepte einlegt, woraufhin das Rezept zu einem Rezeptscanner gelangt. Unmittelbar nach Eingabe des Rezepts wird der Zugriff auf den Einzugsschacht 6 verriegelt, so dass der Kunde 3 das Rezept nicht ohne Zustimmung des Apothekers 17 wieder entnehmen kann. Außerdem wird das Arzneimittel-Ausgabefach 7 des Terminals 2 verriegelt. Möglicherweise noch darin enthaltene Packungen oder andere Objekte werden aus dem Fach 7 entfernt. Anschließend wird das Rezept unter Steuerung durch die Server-Software gescannt, wobei ein erstes elektronisches Abbild des Rezepts erzeugt wird. Das elektronische Abbild des Rezepts wird dann von der Server-Software über die oben beschriebene Datenkommunikationsverbindung zu der Apotheker-Anwendung auf dem Rechner 16 übertragen und anschließend auf dem Bildschirm des Computers 16 angezeigt, so dass der Apotheker 17 das angezeigte Rezept prüfen kann. Anschließend nimmt der Apotheker 17 über die Videokonferenz-Einrichtungen (Konferenz-Software und Videokameras, Bildschirme, Mikrofone und Lautsprecher) Kontakt zu dem Kunden 3 auf, wobei er den Kunden berät und dabei eine Abstimmung über die auszugebenden Arzneimittelpackungen ausführt. Nachdem der Apotheker 17 anhand des Rezepts und in Absprache mit dem Kunden 3 die auszugebenden Arzneimittelpackungen festgelegt hat, gibt er die Auswahl der auszugebenden Arzneimittelpackungen in die Apotheker-Anwendung ein. Dabei kann der Apotheker anhand von Informationen, die ihm von dem Kommissionierautomaten 4 übermittelt worden sind, die Verfügbarkeit der gewünschten Arzneimittelpackungen prüfen, so dass er nur solche Arneimittelpackungen anfordert, die auch im Kommissionierautomaten 4 vorrätig sind. Die Apotheker-Anwendung übermittelt dann eine Anforderung der auszugebenden Packungen an den Kommissionierautomaten 4 und die Server-Software des Terminals 2. Diese tragen dann dafür Sorge, dass die angeforderten Packungen in das Ausgabefach 7 des Terminals 2 transportiert werden. Sofern mehrere Arzneimittelpackungen ausgegeben werden sollen, könnte das Verfahren der Übermittlung der auszugebenden Packungen durch den Apotheker an die Apotheker-Anwendung, die Weitergabe der Informationen an den Kommissionierautomaten 4 und der Transport der auszugebenden Packung in das Ausgabefach 7 auch schrittweise Packung für Packung ausgeführt werden. Nach Abschluss des Transports der auszugebenden Arzneimittelpackungen (bei einer alternativen Ausführungsform auch bereits zuvor) wird ein Livebild der im Ausgabefach liegenden Arzneimittelpackungen an den Apotheker übermittelt, der anhand dieses Bildes eine Kontrolle der Abgabe durchführt. Anstelle eines Livebilds können auch mehrere Livebilder aus verschiedenen Perspektiven an die Apotheker-Anwendung übermittelt und auf dem Bildschirm des Apothekerrechners 16 gleichzeitig oder wahlweise angezeigt werden. Sobald sich der Apotheker davon überzeugt hat, dass die richtigen Arzneimittelpackungen im Ausgabefach 7 einliegen und soweit ein Bezahlen der Arzneimittel erforderlich ist, gibt er den Bezahlvorgang frei, wobei er den Kunden per Videokonferenz auffordert, die Arzneimittelpackungen zu bezahlen. Der Bezahlvorgang kann dann mit Hilfe der Eingabe von Münzen oder Geldscheinen oder mit Hilfe des Eingebens einer Geldkarte oder Kreditkarte einschließlich der Eingabe einer PIN ausgeführt werden. Sobald die Bezahlung der geforderten Beträge durch die Zahlungskomponenten bestätigt wurde, weist die Server-Software den Rezeptdrucker an, das Rezept mit Informationen über die Abgabe von Arzneimittelpackungen sowie mit einem ersten eindeutigen Identifizierer zu bedrucken. Der Identifizierer kann beispielsweise ein eindeutiger und einmaliger Code (beispielsweise Zahlencode oder alphanumerischer Code) sein. Sobald das Rezept mit den Angaben bedruckt worden ist, wird ein zweites elektronisches Abbild des Rezepts mit Hilfe des Rezeptscanners erzeugt. Aus diesem zweiten elektronischen Abbild wird ein elektronisches Dokument, beispielsweise eine PDF-Datei, erzeugt, wobei das elektronische Dokument mit einem zweiten Identifizierer verbunden wird, der dem ersten Identifizierer eindeutig zugeordnet ist. Beispielsweise wird als zweiter Identifizierer einfach der Dateiname des elektronischen Dokuments verwendet, wobei der Dateiname wiederum den Code des ersten Identifizierers enthält.

Das weitere Verfahren wird anhand der Figur 2B beschrieben. Nachdem das PDF-Dokument, das aus dem elektronischen Abbild des mit dem ersten Identifizierer bedruckten Rezepts gewonnen wurde, auf der Festplatte des Servers im Terminal 2 abgelegt worden ist, signalisiert der Server der Apotheker-Anwendung, dass das Rezept bereit zum Signieren ist. Die Apotheker-Anwendung übermittelt der Signatursoftware den Dateipfad, unter dem das zu signierende PDF-Dokument abgelegt ist, sowie einen weiteren Dateipfad, unter dem das signierte Dokument abgelegt werden soll. Die Dateinamen des noch nicht signierten und des signierten Dokuments bleiben gleich. Die beiden Versionen unterscheiden sich nur hinsichtlich des Ablageorts auf der Festplatte des Servers des Terminals 2. Bei alternativen Ausführungsformen des Verfahrens könnten sich die Dateinamen auch unterscheiden und in demselben Verzeichnis abgelegt werden. Die Dateien brauchen auch nicht zwangsläufig im Server des Terminals 2 abgelegt zu werden; sie könnten auch in einem anderen sicheren Speicher gespeichert werden. Nach dem Übermitteln des Speicherorts (Dateipfads) findet unter der Kontrolle der Signatursoftware das Signieren des PDF-Dokuments statt. Dazu wird das PDF-Dokument von der Festplatte des Servers geladen und angezeigt, woraufhin der Apotheker das Rezeptabbild prüft. Nachdem der Apotheker eingegeben hat, dass das Rezeptabbild in Ordnung ist, wird eine Authentifizierung gestartet. In deren Rahmen muss der Apotheker 17 seine Signaturkarte 19 in das Lesegerät 18 einlegen. Anschließend wird er aufgefordert, eine PIN einzugeben. Sobald die korrekte PIN eingegeben ist, signalisiert der Kartenleser, dass die Authentifizierung in Ordnung ist, woraufhin die Signatursoftware die Signatur ausführt und die signierte PDF-Datei an dem vorgegebenen Ort (Verzeichnis gemäß Dateipfad) auf der Festplatte des Servers ablegt. Dann meldet die Signatursoftware der Apotheker-Anwendung den Abschluss der Signatur. Anschließend meldet die Apotheker-Anwendung dem Server den Abschluss der Signatur, woraufhin dieser das Ausgabefach 7 entriegelt, also freigibt. Dem Kunden wird angezeigt, dass er die Arzneimittel entnehmen kann. Sobald die Meldung "Ausgabefach freigegeben" an die Server-Software ausgegeben wurde, übermittelt diese ein Livebild des Ausgabefachs 7 an die Apotheker-Anwendung, die für eine Anzeige des Livebilds des Ausgabefachs 7 auf der Anzeigevorrichtung des Apothekerrechners 16 sorgt. Die Server-Software bestätigt dann die Bezahlung an die Zahlungskomponenten. Außerdem entnimmt der Kunde 3 die Arzneimittel aus dem Ausgabefach 7. Dies erkennt der Apotheker 17 anhand des übermittelten Livebilds, woraufhin er nach vollständiger Entnahme der Arzneimittelpackungen aus dem Ausgabefach eine entsprechende Rückmeldung an die Apotheker-Anwendung eingibt. Dieser Abgabestatus wird von der Apotheker-Anwendung an die Server-Software übermittelt. Diese Server-Software sorgt anschließend dafür, dass der Abgabestatus auf das Rezept aufgedruckt und das bedruckte Rezept sicher abgelegt wird. Nach dem Aufdrucken wird das Rezept in das Rezept-Ablagefach transportiert. Der Abschluss des Transports wird der Server-Software angezeigt, welche daraufhin die Meldung "Warenausgabe erfolgt" an die Apotheker-Anwendung weitergibt, die dann eine entsprechende Meldung dem Apotheker anzeigt, dass der Vorgang abschlossen ist.

Es gibt eine Reihe von Routinen, die auf auftretende Fehler oder andere vom oben geschilderten normalen Verfahren abweichende Zustände reagieren können. Beispielsweise ist es möglich, dass nicht sämtliche auf dem Rezept verschriebene Arzneimittelpackungen in dem Kommissionierautomaten 4 verfügbar sind oder durch dort verfügbare Arzneimittelpackungen substituiert werden können. Dies wird dem Apotheker angezeigt, woraufhin er entweder den Verkaufsvorgang abbrechen und dem Kunden das Rezept zurückgeben kann oder er die verfügbaren Arzneimittelpackungen abgeben kann. Entscheidet sich der Apotheker dafür, nur einen Teil der auf dem Rezept aufgedruckten Arzneimittelpackungen abzugeben, so wird das oben geschilderte Verfahren etwas modifiziert. In die signierte PDF-Datei wird ein zusätzlicher Abschnitt aufgenommen, der die nicht abgegebenen Rezeptpositionen kennzeichnet. Zusätzlich enthält die signierte PDF-Datei einen Hinweis "Folgeabgabe durch händische Signatur auf dem Papierrezept". Auf das Papierrezept wird der Abgabestatus "Teilmenge" vor Ablage des Rezepts aufgedruckt. Wenn später die restlichen Arzneimittel des Rezepts ausgegeben werden, muss dies bei dieser Ausführungsform manuell auf dem Papierrezept vermerkt und abgezeichnet werden.

Ein anderer Fall ist der Abbruch des Verfahrens vor erfolgter Bezahlung. Sofern der Verkauf eines rezeptpflichtigen Medikaments vor dem Abschluss des Bezahlvorgangs abgebrochen wird, das heißt vor dem Bedrucken des Rezepts, so kann das Rezept an den Kunden zurückgegeben werden. Bereits bezahltes Bargeld wird dem Kunden zurückgegeben, begonnene Kartenzahlungstransaktionen werden abgebrochen. Die bereits in das Ausgabefach 7 transportierten Arzneimittelpackungen werden aus dem Ausgabefach entleert und können beispielsweise wieder in den Kommissionierautomaten 4 eingelagert werden.

Es kann auch der Fall eintreten, dass die Signatur nicht erfolgreich ist. In diesem Fall wird zunächst üblicherweise der Signaturvorgang wiederholt. Wenn die Signatur endgültig fehlschlägt, signalisiert die Apotheker-Anwendung auf dem Computer 16 der Server-Software den Abbruch des Verkaufsvorgangs. Der Kunde erhält bereits bezahltes Bargeld zurück, eine Kartenzahlung wird ggf. storniert. Allerdings kann das Rezept wegen der bereits durchgeführten Bedruckung jetzt nicht an den Kunden zurückgegeben werden. Stattdessen wird es mit dem Status "Keine Signatur" bedruckt und in das Ablagefach transportiert. Währenddessen kann der Apotheker den Kunden über Videokonferenz über die weitere Vorgehensweise informieren. Das bereits gefüllte Ausgabefach wird entleert.

Anstelle der oben beschriebenen gegenwärtig bevorzugten Verfahrensabläufe sind eine Vielzahl von Alternativen im Rahmen der beanspruchten Verfahren denkbar.

## Patentansprüche

1. Verfahren zum Auslösen einer durch ein Papierdokument autorisierten Aktion einer Vorrichtung, wobei die Aktion ein eindeutiges Zuordnen einer Signatur zu dem Papierdokument voraussetzt, wobei:
a) an einem ersten Ort, an dem die Aktion ausgeführt werden soll, das Papierdokument in eine Aufnahme eingelegt, von einer Aufbringvorrichtung ein erster eindeutiger Identifizierer auf das Papierdokument unlösbar aufgebracht und ein elektronisches Abbild des Papierdokuments erzeugt wird, wobei dem ersten Identifizierer ein zweiter Identifizierer eindeutig zugeordnet wird und aus dem elektronischen Abbild ein elektronisches Dokument erzeugt und untrennbar mit dem zweiten Identifizierer verbunden wird,
b) ein Abbild des elektronischen Dokuments auf einer Ausgabevorrichtung einer an einem zweiten, entfernten Ort befindlichen Datenverarbeitungseinrichtung einer zum Signieren autorisierten Person angezeigt und das elektronische Dokument von der autorisierten Person durch eine vorgegebene Eingabe in eine Eingabevorrichtung elektronisch signiert wird,
c) das mit dem zweiten Identifizierer verbundene und signierte elektronische Dokument in einem abgesicherten Speicher gespeichert wird, und
d) die autorisierte Aktion durch die Vorrichtung ausgeführt wird, wenn das signierte elektronische Dokument gespeichert worden ist,
wobei im Schritt a) das elektronische Abbild des Papierdokuments erstellt wird, nachdem der erste Identifizierer auf dem Papierdokument unlösbar aufgebracht worden ist, wobei das elektronische Abbild, aus dem das elektronische Dokument erzeugt wird, ein Abbild zumindest eines Teils des Papierdokuments mit dem aufgebrachten ersten Identifizierer umfasst.

2. Verfahren nach Anspruch 1, **dadurch gekennzeichnet, dass** der erste Identifizierer einen alphanumerischen Code umfasst, wobei der zweite Identifizierer dem ersten Identifizierer eindeutig zugeordnet und mit dem elektronischen Dokument verbunden wird, indem ein den alphanumerischen Code enthaltender Dateiname des elektronischen Dokuments als zweiter Identifizierer verwendet wird.

3. Verfahren nach einem der Ansprüche 1 - 2, **dadurch gekennzeichnet, dass**
vor dem Aufbringen des ersten eindeutigen Identifizierers auf das Papierdokument im Schritt a) das Papierdokument in eine Aufnahme eingelegt und danach der Zugriff auf das Papierdokument gesperrt wird,
wobei dann, wenn das Verfahren vor dem Ausführen der Aktion oder auch nur eines Teils der Aktion abgebrochen wurde, der Zugriff auf das Papierdokument zur Entnahme wieder freigegeben wird,
anderenfalls nach vollständigem oder teilweisem Ausführen der Aktion das Papierdokument in einer gegen unbefugten Zugriff gesicherten Ablage abgelegt wird.

4. Verfahren nach Anspruch 3, **dadurch gekennzeichnet, dass** das Papierdokument vor dem Ablegen in die gegen unbefugten Zugriff gesicherte Ablage mit einem das vollständige Ausführen der Aktion bzw. den ausgeführten Teil der Aktion kennzeichnenden Aufdruck versehen wird.

5. Verfahren nach einem der Ansprüche 1 - 4, **dadurch gekennzeichnet, dass** das Papierdokument Angaben enthält, die die Art der autorisierten Aktion definieren, und dass vor dem Schritt a)
ein erstes elektronisches Abbild des Papierdokuments erstellt wird,
auf der Basis des ersten elektronischen Abbilds die von den Angaben definierte Art der Aktion bestimmt wird,
ermittelt wird, ob es möglich ist, die definierte Art der Aktion an dem ersten Ort auszuführen, und
nur dann, wenn dies der Fall ist, das Verfahren mit dem Schritt a) fortgesetzt wird,
wobei das in Schritt a) erzeugte elektronische Abbild, aus dem das elektronische Dokument erzeugt wird, ein zweites elektronisches Abbild ist.

6. Verfahren nach Anspruch 5, **dadurch gekennzeichnet, dass** auf der Basis des ersten elektronischen Abbilds die von den Angaben definierte Art der Aktion bestimmt wird, indem die Angaben mittels eines Texterkennungsprogramms ermittelt werden und automatisch ermittelt wird, ob es möglich ist, die definierte Art der Aktion auszuführen.

7. Verfahren nach Anspruch 5, **dadurch gekennzeichnet, dass** auf der Basis des ersten elektronischen Abbilds die von den Angaben definierte Art der Aktion bestimmt wird, indem das Abbild sowie Angaben zu ausführbaren Aktionen auf der Ausgabevorrichtung der an dem zweiten Ort befindlichen Datenverarbeitungseinrichtung der autorisierten Person angezeigt werden und die autorisierte Person in Abhängigkeit davon die definierte Art der Aktion durch entsprechende Eingaben vorgibt.

8. Verfahren nach einem der Ansprüche 1 - 7, **dadurch gekennzeichnet, dass** das Papierdokument ein Arzneimittel-Rezept und die autorisierte Aktion eine Abgabe von auf dem Rezept verzeichneten Arzneimittelpackungen an einen Kunden an dem ersten Ort durch eine Abgabevorrichtung ist, wobei das Abbild des elektronischen Dokuments an dem zweiten, entfernten Ort einem Apotheker als der zum Signieren berechtigten Person angezeigt wird.

9. Verfahren nach Anspruch 8, **dadurch gekennzeichnet, dass** im Schritt a):
a1) von der Aufnahmevorrichtung an dem ersten Ort ein erstes elektronisches Abbild des Rezepts erzeugt und auf der Ausgabevorrichtung der an dem zweiten, entfernten Ort befindlichen Datenverarbeitungseinrichtung dem Apotheker angezeigt wird,
a2) anschließend geprüft wird, ob eine Abgabe der auf dem Rezept verzeichneten Arzneimittelpackungen durch einen an dem ersten Ort befindlichen Abgabeautomaten möglich ist,
a3) sofern diese Abgabe möglich ist, durch eine entsprechende Eingabe des Apothekers ein Transport der abzugebenden Arzneimittelpackungen zu einem Ausgabefach angewiesen wird, wobei der Zugriff des Kunden auf das Ausgabefach gesperrt wird,
a4) dann mittels Erfassungseinrichtungen an dem ersten Ort geprüft wird, ob weitere Voraussetzungen für die Abgabe der Arzneimittelpackungen erfüllt sind,
a5) sofern die weiteren Voraussetzungen erfüllt sind, von der Aufbringvorrichtung der erste eindeutige Identifizierer auf das Rezept aufgebracht und von der Aufnahmevorrichtung ein zweites elektronisches Abbild des Rezepts erzeugt wird, und
a6) aus dem zweiten elektronischen Abbild das elektronische Dokument erzeugt und mit dem zweiten Identifizierer untrennbar verbunden wird, und
dass im Schritt d) der Zugriff des Kunden auf das Ausgabefach freigegeben wird.

10. Verfahren nach Anspruch 9, **dadurch gekennzeichnet, dass** im Schritt a4) mittels einer auf das Ausgabefach gerichteten Kamera ein Bild der in das Ausgabefach transportierten Arzneimittelpackungen erzeugt und als weitere Voraussetzung für die Abgabe der Arzneimittelpackungen anhand des Bildes geprüft wird, ob sich die richtigen Arzneimittelpackungen im Ausgabefach befinden.

11. Verfahren nach Anspruch 9 oder 10, **dadurch gekennzeichnet, dass** im Schritt a4) eine Aufforderung für einen Bezahlvorgang an den Kunden ausgegeben und als weitere Voraussetzungen für die Abgabe der Arzneimittelpackungen geprüft wird, ob der Bezahlvorgang erfolgreich abgeschlossen wurde.

12. Verfahren nach einem der Ansprüche 9 - 11, **dadurch gekennzeichnet, dass** im Schritt d) geprüft wird, ob sämtliche Arzneimittelpackungen vollständig aus dem freigegebenen Ausgabefach entnommen worden sind, und dann, wenn dies nicht der Fall ist, ein Signal an den Kunden ausgegeben wird.

13. Verfahren nach einem der Ansprüche 9 - 12, **dadurch gekennzeichnet, dass** im Schritt d) geprüft wird, ob sämtliche Arzneimittelpackungen vollständig aus dem freigegebenen Ausgabefach entnommen worden sind, und dann, wenn dies der Fall ist, das Papierdokument mit einem das vollständige Ausführen der autorisierten Aktion kennzeichnenden Aufdruck versehen wird, anderenfalls das Papierdokument mit einem die unvollständige Entnahme der Arzneimittelpackungen kennzeichnenden Aufdruck versehen wird.

## Claims

1. A method for triggering an action of a device, which action is authorised by a paper document, wherein the action requires that a signature is unequivocally assigned to the paper document, wherein:
a) at a first location, at which the action is to be executed, the paper document is inserted into a holder, a first unequivocal identifier is insolubly applied by an application device to the paper document and an electronic image of the paper document is generated, wherein a second identifier is unequivocally assigned to the first identifier and an electronic document is generated from the electronic image and is inseparably connected with the second identifier,
b) an image of the electronic document is displayed on an output device of a data processing means at a second remote location of a person authorised to sign and the electronic document is electronically signed by the authorised person by means of a specified input into an input device,
c) the signed electronic document connected with the second identifier is stored in a secure memory, and
d) the authorised action is executed by the device, after the signed electronic document has been stored,
wherein in step a) the electronic image of the paper document is created after the first identifier has been insolubly applied to the paper document, wherein the electronic image, from which the electronic document is generated, comprises an image of at least one part of the paper document with the applied first identifier.

2. The method according to claim 1, **characterised in that** the first identifier comprises an alpha-numeric code, wherein the second identifier is unequivocally assigned to the first identifier and connected with the electronic document by using a file name of the electronic document containing the alphanumeric code as the second identifier.

3. The method according to one of claims 1 - 2, **characterised in that**
prior to applying the first unequivocal identifier to the paper document in step a) the paper document is inserted into a holder and thereafter access to the paper document is blocked,
wherein then, when the process has been aborted prior to executing the action or just a part of the action, access to the paper document for removal is again allowed,
otherwise, after the action has been fully or partially executed, the paper document is filed in a place secured against unauthorised access.

4. The method according to claim 3, **characterised in that** the paper document, prior to filing it in the place secured against unauthorised access, is provided with an imprint signifying full or partial completion of the action.

5. The method according to one of claims 1 - 4, **characterised in that** the paper document contains details defining the type of authorised action, and **in that** prior to step a)
a first electronic image of the paper document is created,
the type of action defined by the details is determined on the basis of the first electronic image,
it is ascertained as to whether it is possible to execute the defined type of action at the first location, and
only if this is the case, the process is continued with step a),
wherein the electronic image generated in step a) from which the electronic document is generated, is a second electronic image.

6. The method according to claim 5, **characterised in that** the type of action defined by the details is determined on the basis of the first electronic image by ascertaining the details by means of a text recognition program and by automatically ascertaining, as to whether it is possible to execute the defined type of action.

7. The method according to claim 5, **characterised in that** the type of action defined by the details is determined on the basis of the first electronic image in that the image as well as details of the actions to be executed are displayed on the output device of the data processing means of the authorised person at the second location, and the authorised person, in dependence thereof, specifies the defined type of action by making respective inputs.

8. The method according to one of claims 1 - 7, **characterised in that** the paper document is a prescription for medicines and the authorised action is the dispensing of packs of medicines specified on the prescription to a customer at the first location by a dispensing device, wherein the image of the electronic document is displayed to a pharmacist as the person authorised to sign, at the second remote location.

9. The method according to claim 8, **characterised in that** in step a) :
a1) a first electronic image of the prescription is generated by the holding device at the first location, and is displayed to the pharmacist on the output device of the data processing device at the second remote location,
a2) a check is then carried out, whether the dispensing of the packs of medicines specified on the prescription by an automatic dispensing machine at the first location is possible,
a3) insofar as this dispensing is possible, a corresponding input by the pharmacist is made to initiate transport of the packs of medicines to an output tray, wherein access by the customer to the output tray is blocked,
a4) then a check is carried by means of recording means at the first location, whether further requirements for the dispensing of the packs of medicine have been met,
a5) insofar as the further requirements have been met, the first unequivocal identifier is applied to the prescription by the application device and a second electronic image of the prescription is generated by the holding device, and
a6) the electronic document is generated from the second electronic image and is inseparably connected with the second identifier, and
**in that** in step d) access by the customer to the output tray is allowed.

10. The method according to claim 9, **characterised in that** in step a4) a camera directed at the output tray takes a picture of the packs of medicines transported to the output tray and, as a further requirement for dispensing the packs of medicines, a check is carried out by means of the picture, as to whether the correct packs of medicines are in the output tray.

11. The method according to claim 9 or 10, **characterised in that** in step 4a) a request for payment is issued to the customer, and as a further requirement for dispensing the packs of medicines a check is carried out, as to whether the payment process has been successfully completed.

12. The method according to one of claims 9 - 11, **characterised in that** it is checked in step d), as to whether all packs of medicines have been completely removed from the released output tray, and that, if this is not the case a signal is issued to the customer.

13. The method according to one of claims 9 - 12, **characterised in that** it is checked in step d), as to whether all packs of medicines have been completely removed from the released output tray, and that, if this is the case, the paper document is provided with an imprint signifying that execution of the authorised action has been completed, otherwise the paper document is provided with an imprint signifying that removal of the packs of medicines is incomplete.

## Revendications

1. Procédé pour le déclenchement d'une action d'un dispositif autorisée par un document papier, dans lequel l'action a pour condition une association univoque d'une signature au document papier, dans lequel :
a) à un premier endroit où l'action doit être exécutée, le document papier est déposé dans un réceptacle, un dispositif d'application appliquant de manière indélébile un premier identifiant univoque sur le document papier et une représentation électronique du document papier étant générée, dans lequel on associe de manière univoque un deuxième identifiant au premier identifiant et un document électronique étant généré à partir de la représentation électronique et étant lié de manière inséparable au deuxième identifiant,
b) une représentation du document électronique étant affichée sur un dispositif de sortie d'un dispositif de traitement de données se trouvant à un deuxième endroit distant pour une personne habilitée à signer, et le document électronique étant signé électroniquement par la personne habilitée grâce à une saisie prescrite dans un dispositif de saisie,
c) le document électronique en lien avec le deuxième identifiant et signé étant enregistré dans une mémoire sécurisée, et
d) l'action autorisée étant exécutée par le dispositif lorsque le document électronique signé a été enregistré,
dans lequel, à l'étape a), la représentation électronique du document papier est créée une fois que le premier identifiant a été appliqué de manière indélébile sur le document papier, dans lequel la représentation électronique à partir de laquelle le document électronique est généré comprend une représentation d'au moins une partie du document papier avec le premier identifiant appliqué dessus.

2. Procédé selon la revendication 1, **caractérisé en ce que** le premier identifiant comprend un code alphanumérique, dans lequel le deuxième identifiant est associé de manière univoque au premier identifiant et est lié au document électronique **en ce qu'**un nom de fichier du document électronique contenant le code alphanumérique est utilisé en tant que deuxième identifiant.

3. Procédé selon l'une des revendications 1 - 2, **caractérisé en ce que**
avant l'application du premier identifiant univoque sur le document papier, à l'étape a), le document papier est déposé dans un réceptacle et l'accès au document papier étant ensuite bloqué,
dans lequel, lorsque le procédé a été interrompu avant l'exécution de l'action ou de seulement une partie de l'action, l'accès au document papier est de nouveau débloqué pour le prélèvement,
sinon, après l'exécution complète ou partielle de l'action, le document papier étant déposé dans un emplacement de dépôt protégé contre un accès non autorisé.

4. Procédé selon la revendication 3, **caractérisé en ce que**, avant le dépôt à l'emplacement de dépôt protégé contre un accès non autorisé, le document papier est doté d'une impression caractérisant l'exécution complète de l'action ou de la partie de l'action exécutée.

5. Procédé selon l'une des revendications 1 - 4, **caractérisé en ce que** le document papier contient des indications qui définissent le type de l'action autorisée, et **en ce qu'**avant l'étape a)
une première représentation électronique du document papier est créée,
sur la base de la première représentation électronique, le type de l'action défini par les indications étant déterminé,
**en ce que** l'on détermine s'il est possible d'exécuter le type défini de l'action au premier endroit, et
seulement si cela est le cas, le procédé se poursuivant avec l'étape a),
dans lequel la représentation électronique générée à l'étape a), à partir de laquelle le document électronique est généré, est une deuxième représentation électronique.

6. Procédé selon la revendication 5, **caractérisé en ce que**, sur la base de la première représentation électronique, le type de l'action défini par les indications est déterminé **en ce que** les indications sont déterminées au moyen d'un programme de reconnaissance de texte et **en ce que** l'on détermine automatiquement s'il est possible d'exécuter le type défini de l'action.

7. Procédé selon la revendication 5, **caractérisé en ce que**, sur la base de la première représentation électronique, le type de l'action défini par les indications est déterminé **en ce que** la représentation ainsi que des indications pour des actions exécutables sont affichées sur le dispositif de sortie du dispositif de traitement de données se trouvant au deuxième endroit pour la personne habilitée et **en ce que** la personne habilitée prescrit, en fonction de cela, le type défini de l'action grâce à des saisies correspondantes.

8. Procédé selon l'une des revendications 1 - 7, **caractérisé en ce que** le document papier est une ordonnance de médicaments et l'action autorisée une délivrance d'emballages de médicaments indiqués sur l'ordonnance à l'attention d'un client au premier endroit grâce à un dispositif de délivrance, dans lequel la représentation du document électronique est affichée au deuxième endroit distant pour un pharmacien en tant que personne habilitée à signer.

9. Procédé selon la revendication 8, **caractérisé en ce qu'**à l'étape a):
a1) le dispositif de réception au premier endroit génère une première représentation électronique de l'ordonnance et celle-ci étant affichée sur le dispositif de sortie du dispositif de traitement de données se trouvant au deuxième endroit distant pour le pharmacien,
a2) **en ce que** l'on vérifie ensuite si une délivrance des emballages de médicaments indiqués sur l'ordonnance est possible grâce à un distributeur automatique se trouvant au premier endroit,
a3) dans la mesure où cette délivrance est possible, grâce à une saisie correspondante du pharmacien, un transport des emballages de médicaments à délivrer vers un bac de sortie étant ordonné, dans lequel l'accès au bac de sortie est bloqué pour le client,
a4) **en ce que** l'on vérifie ensuite, au moyen de dispositifs de détection au premier endroit, si d'autres conditions préalables pour la délivrance des emballages de médicaments sont satisfaites,
a5) dans la mesure où les autres conditions préalables sont satisfaites, le dispositif d'application appliquant le premier identifiant univoque sur l'ordonnance et le dispositif de réception générant une deuxième représentation électronique de l'ordonnance, et
a6) **en ce que** l'on génère, à partir de la deuxième représentation électronique, le document électronique et le lie de manière inséparable au deuxième identifiant, et
**en ce qu'**à l'étape d), l'accès au bac de sortie est débloqué pour le client.

10. Procédé selon la revendication 9, **caractérisé en ce qu'**à l'étape a4), on génère au moyen d'une caméra orientée vers le bac de sortie une image des emballages de médicaments transportés dans le bac de sortie et **en ce que** l'on vérifie à l'aide de l'image en tant qu'une autre condition préalable pour la délivrance des emballages de médicaments, si les bons emballages de médicaments se trouvent dans le bac de sortie.

11. Procédé selon la revendication 9 ou 10, **caractérisé en ce qu'**à l'étape a4), on fait sortir une demande pour un processus de paiement à l'attention du client et **en ce qu'**en guise d'une autre condition préalable pour la délivrance des emballages de médicaments, on vérifie si le processus de paiement s'est clos avec succès.

12. Procédé selon l'une des revendications 9 - 11, **caractérisé en ce qu'**à l'étape d), on vérifie si l'ensemble des emballages de médicaments ont été entièrement prélevés du bac de sortie débloqué, et lorsque ceci n'est pas le cas, un signal étant alors émis à l'attention du client.

13. Procédé selon l'une des revendications 9 - 12, **caractérisé en ce qu'**à l'étape d), on vérifie si l'ensemble des emballages de médicaments ont été entièrement prélevés du bac de sortie débloqué, et lorsque ceci est le cas, le document papier étant alors doté d'une impression caractérisant l'exécution complète de l'action autorisée, sinon, le document papier étant doté d'une impression caractérisant le prélèvement incomplet des emballages de médicaments.
